# EUROPEAN PATENT APPLICATION

(11) **EP 4 659 768 A1**
(43) Date of publication of application: **10.12.2025**
(21) Application number: 24179644.0
(22) Date of filing: 03.06.2024
(51) Int. Cl.: A61L 9/04, A61L 9/14, B05B 12/08, G06F 8/00, G06F 9/00, H04S 1/00, H04R 1/00

(54) **MULTISENSORY SYSTEM**

(71) Applicant: Enstase SA, 1003 Lausanne (CH)
(72) Inventor: MONIN, Fabien, 1003 Lausanne (CH)
(74) Representative: Micheli & Cie SA

(57) **Abstract**

System for creating a multisensory environment, said system comprising a fragrance atomizer (2) and a computing device (1), the fragrance atomizer (2) comprising sprayer (22) for spraying upon actuation a fragrant liquid contained in the fragrance atomizer (2), the fragrance atomizer (2) further comprising a switch (23) and a wireless communication interface (20), wherein the switch (23) is configured to be actuated upon actuation of the sprayer (22) and to emit upon actuation over the wireless communication interface (20) a signal representative of the actuation of the sprayer (22); the computing device (1) comprising a wireless communication interface (10) for communicating with the wireless communication interface (20) of the fragrance atomizer (2) and a sound player for playing a sound upon reception by the wireless communication interface (10) of the signal representative of the actuation of the sprayer (22).

## Description

The present invention relates to a system for creating a multisensory environment. The present invention relates in particular to a system for creating a personalized multisensory environment for a specific user.

Stimuli from the environment, in particular smells and sounds, may largely influence one's mood, liveliness and/or ability to concentrate. Some stimuli may have a positive influence for example by creating a comfortable environment while some may have a negative influence for example through disturbing noises and/or unpleasant smells. Most of the stimuli to which one is confronted on a daily basis are usually beyond one's control and may for example include noises from other people's activities such as traffic noise, music, bursts of voices, etc. Being exposed to unwanted and possibly disturbing stimuli may significantly impact one's overall wellbeing. There is thus a need for means to provide at least temporary relief from these stimuli by creating a positive environment, preferably adapted to one's current state of mind.

An aim of the present invention is thus to provide a system and a method for creating a personalized multisensory environment.

Another aim of the present invention is to provide a system and a method for creating a multisensory environment with appropriate properties for positively influencing a user's wellbeing.

Still another aim of the present invention is to provide a system and a method for creating a multisensory environment that is adapted to a user's mood and/or to the time of the day.

These aims and other advantages are achieved by a system for creating a multisensory environment, said system comprising a fragrance atomizer and a computing device, the fragrance atomizer comprising a sprayer for spraying upon actuation a fragrant liquid contained in the fragrance atomizer, the fragrance atomizer further comprising a switch and a wireless communication interface, wherein the switch is configured to be actuated upon actuation of the sprayer and to emit upon actuation over the wireless communication interface a signal representative of the actuation of the sprayer; the computing device comprising a wireless communication interface for communicating with the wireless communication interface of the fragrance atomizer and a sound player for playing a sound upon reception by the wireless communication interface of the signal representative of the actuation of the sprayer.

The system of the invention thus allows creating a multisensory environment for the user by the quasi simultaneous diffusion of fragrance and sound that is adapted to the user's mood and/or personal purpose of the day. The thus created multisensory environment may contribute in enhancing the user's mood and/or creating a stimulating ambiance in relation with the user's plans or personal purpose of the day.

The computing device preferably further comprises at least one input interface for receiving one or more inputs from a user of the computing device. The at least one input interface is for example one of a microphone, a keyboard, a touch screen and a camera, or a combination thereof.

The sound played upon reception of the signal representative of the actuation of the sprayer is preferably determined on the basis of the one or more inputs.

In preferred embodiments, the switch is an energy harvesting switch for powering the wireless communication interface of the fragrance atomizer.

The switch and the wireless communication interface of the fragrance atomizer are for example located in the spray nozzle.

The above aims and other advantages are also achieved by a method for creating a multisensory environment with such a system, the method comprising the steps of actuating by a user the sprayer, emitting by the fragrance atomizer, over the wireless communication interface of the fragrance atomizer, a signal indicative of the actuation of the sprayer, receiving by the computing device, over the wireless communication interface of the computing device, the signal indicative of the actuation of the sprayer, playing a sound by the computing device upon reception of the signal indicative of the actuation of the sprayer.

In embodiments, the method further comprises the preliminary steps of receiving by the computing device at least one input from the user indicative of the user's mood and/or personal purpose of the day, analyzing by the computing device the received input for determining the user's mood and/or personal purpose of the day, selecting by the computing device a sound to be played in said step of playing a sound based on the determined user's mood and/or personal purpose of the day.

The user input is for example at least one of a voice sample, a menu selection, a face picture and a text string from the user, or a combination thereof.

The above aims and other advantages are further achieved also by a computer program containing commands for a computing device to perform such a method when the computer program is run on the central processing unit of the computing device.

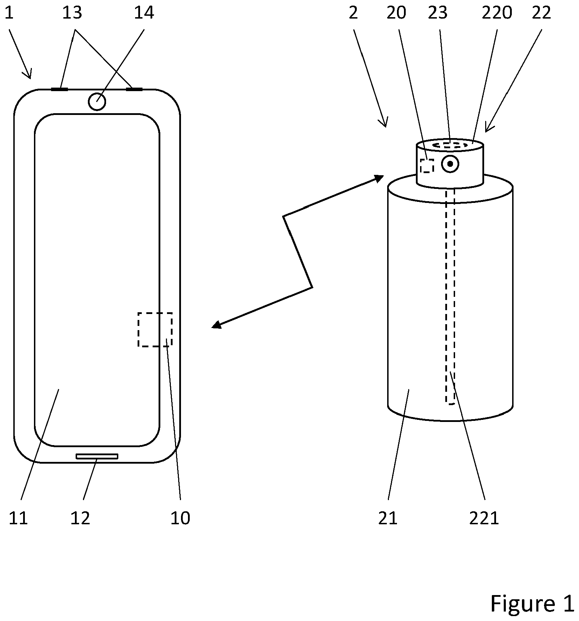

The invention will be better understood by reading the following description, illustrated by the sole figure 1 that schematically shows a system for creating a multisensory environment according to an embodiment of the invention.

Referring to figure 1, the system of the invention comprises a computing device 1, preferably a portable computing device such as for example a mobile phone, a tablet or a laptop computer, and a fragrance atomizer 2 able to wirelessly communicate with the computing device 1. Each of the computing device 1 and the atomizer 2 comprises a wireless communication interface 10, 20 able to wirelessly communicate with each other. The wireless communication interfaces 10, 20 are for example Bluetooth interfaces. Other wireless communication interfaces, preferably near-field wireless communication interfaces, using other communication protocols are however possible within the scope of the invention.

In a known manner, the computing device 1 preferably further comprises a display 11, for example a touch screen, input means such as for example a microphone 12, a camera 14 and/or a keyboard, for example a virtual keyboard that is at least temporarily displayed on the touch screen, and a central processing unit for controlling the wireless communication interface 10, the input means and other components of the computing device and for running computer programs stored on a memory of the computing device 1. According to the invention, the computing device 1 further comprises a sound player preferably in the form of an application or computer program runed by the central processing unit and playing sound, for example music, from a local and/or from an online sound library. The computing device 1 preferably comprises one or more speakers 13 for playing the sound launched by the sound player. Alternatively, or in addition thereof, the computing device 1 is configured in a known manner to play the sound launched by the sound player on a remote speaker or sound system.

The atomizer 2 comprises a bottle 21 for containing a fragrant liquid, and a sprayer 22 closing the bottle 21 for spraying the fragrant liquid out of the bottle. The sprayer is for example a pump spray, an aerosol type sprayer, a sprayer activated by squeezing of flexible walls of the atomizer, or any other appropriated sprayer. In the illustrated exemplary but not limiting embodiment, the sprayer 22 is a pump spray comprising a spray nozzle 220 located on top of the bottle 21, and a tube 221 inside the bottle 21 extending from the spray nozzle 210 to the bottom of the bottle 21. When the nozzle 220 is pressed down, a dose of fragrant liquid is pumped through the tube 221 up to the nozzle 220 and sprayed outside the bottle 21 through the spray nozzle 220.

According to the invention, the atomizer 2 further comprises a switch 23 configured to be activated when the sprayer 22 is activated, for example when the nozzle 220 is pressed down, and an electronic circuit, not illustrated in figure 1, for controlling the wireless communication interface 20. The switch 23 is preferably located inside or on the sprayer 22, for example in the nozzle 220. The electronic circuit is preferably located inside the sprayer 22, for example in the nozzle 220. Alternatively, the electronic circuit is at least partly located in or on the bottle 21. In embodiments, the electronic circuit comprises an electrical power source, for example a small button battery, for powering the electronic circuit and the wireless communication interface. When the switch is activated, an electrical signal is generated by the electronic circuit and transmitted over the wireless communication to the computing device. Preferably, when the sprayer is not activated, for example when the nozzle is in its rest position, the switch is not activated, for example open, and the electronic circuit is preferably not powered, thereby avoiding any power consumption by the electronic circuit when not in use. When the sprayer is activated for spraying some fragrant liquid, for example when the nozzle is pressed down, the switch is activated, for example closed, and the electronic circuit is powered, preferably for a determined period of time, and generates a signal that is transmitted to the computing device over the wireless communication interface.

In preferred embodiments, the switch 23 is an energy harvesting switch and the wireless communication interface 20 of the atomizer 2 is a low energy wireless communication interface, preferably a low power near-field wireless communication interface. The wireless communication interface 20 of the atomizer 2 and the switch 23 are for example part of an energy harvesting Bluetooth low energy switch, i.e. a battery-free Bluetooth switch. In an energy harvesting switch, electrical energy is generated by a generator, typically by an inductive generator, when the switch 23 is mechanically actuated. The electrical energy harvested from the mechanical actuation of the switch 23 is then sufficient to communicate the switch actuation via the wireless interface 20, for example via a Bluetooth Low Energy interface. The actuation of the switch 23 through actuation of the sprayer 22 thus results in the emission over the wireless interface 20 of a signal that is representative of the nozzle actuation.

Other combinations of switch 23 and wireless communication interface 20 using other technologies and/or communication protocols are nevertheless possible within the frame of the invention in order to generate and emit over the wireless communication interface 20 of the atomizer 2 a signal that is representative of the actuation of the switch 23 and hence of the sprayer 22.

The computing device 1 is configured, typically programmed, to receive over its wireless communication interface 10 the signal emitted by the atomizer 2 and thereby remotely detect the actuation of the sprayer 22.

In preferred embodiments, a computer program is stored in the memory storage of the computing device 1 for performing the steps of a method of the invention for creating a multisensory environment when run by a processing unit of the computing device 1, typically by the central processing unit of the computing device 1.

According to embodiments of the invention, the wireless communication interface 10 of the computing device 1 is first activated.

The sprayer 22 of the atomizer 2 is then actuated and a corresponding signal is emitted over the wireless communication interface 20 of the atomizer 2.

The computing device 1 receives the signal emitted by the atomizer 2 and initiates playing a sound, for example one or more of a piece of music, ambient sounds, or any other appropriate sounds, with the sound player, the sound being played for example over the speakers 13 of the computing device 1 and/or over remote wireless or wired speakers.

In preferred embodiments, the sound to be played by the sound player upon reception of the signal indicative of the actuation of the sprayer 22 is personalized for the user of the system of the invention. The sound to be played is for example chosen according to the user's mood and/or objectives for the day.

In order to choose the sound to be played, the method of the invention for example comprises a preliminary step of determining a mood of the user and/or determining his personal purpose of the day.

This step preferably comprises soliciting, receiving and analyzing one or more real-time inputs from the user. In embodiments, the method for example includes asking a user of the system of the invention to shoot a face picture of him- or herself with the computing device's camera 14, recording his or her voice and/or breathing with the computing device's microphone 12 and/or responding in written and/or orally to questions regarding his or her mood and/or personal purpose of the day.

Inputs thus may include, but are not limited to: a recording of the user's voice and/or breathing, a picture of the user's face and/or a text message that the user would have typed on the computing device 1 and/or selected from a menu displayed on the computing device's display screen. Preferably these inputs are captured using the computing device 1 short before the user actuates the sprayer 22 to spray the fragrance contained in the atomizer 2.

The one or more inputs received by the computing device 1 are analyzed to determine a humor or mood of the user and/or his or her personal purpose of the day. The mood of the user is for example determined based on the user's voice, the user's breathing and/or a picture of the user's face. The inputs are analyzed using for example a trained artificial intelligence piece of software that determines the most probable mood of the user using for example the tone of his or her voice, the rhythm and/or intensity of his or her breathing, and/or the traits of his or her face. Additionally or alternatively, the user's personal purpose of the day may be determined based on written and/or oral information received from the user, for example through a text message, a user's choice in a menu and/or a voice recording of the user.

Preferably, the sound to be played by the computing device 1 is chosen according to the previously determined mood and/or purpose. The sound to be played is for example one or more of pieces of music, sounds of nature and/or animals, etc The method for example includes choosing within a predetermined set of sounds, the sound that is best adapted to the user's mood and/or personal purpose of the day. The predetermined set of sounds is for example determined based on the user's tastes and is a subset of his or her locally stored and/or online musical library and/or other sound library. Additionally or alternatively, the predetermined set of sounds for example includes sounds that are known for their abilities to cure or enhance a mood, stimulate a listener's energy, etc.

Once the sound to be played is determined, it is played by the sound player of the computing device 1 upon reception of the signal indicative of the actuation of the sprayer 22 of the atomizer 2. A multisensory environment is thus created with the fragrance and the sound surrounding the user. The sound being personalized for the user, the multisensory environment will be perceived as positive and preferably stimulating. Creating the multisensory environment according to the invention in the morning, for example, may help a user of the system enhancing his or her mood and motivation for the day to come.

## Claims

1. System for creating a multisensory environment, said system comprising a fragrance atomizer (2) and a computing device (1),
the fragrance atomizer (2) comprising a sprayer (22) for spraying upon actuation a fragrant liquid contained in the fragrance atomizer (2), the fragrance atomizer (2) further comprising a switch (23) and a wireless communication interface (20), wherein the switch (23) is configured to be actuated upon actuation of the sprayer (22) and to emit upon actuation over the wireless communication interface (20) a signal representative of the actuation of the sprayer (22);
the computing device (1) comprising a wireless communication interface (10) for communicating with the wireless communication interface (20) of the fragrance atomizer (2) and a sound player for playing a sound upon reception by the wireless communication interface (10) of the signal representative of the actuation of the sprayer (22).

2. System according to the preceding claim, the computing device (1) further comprising at least one input interface (11, 12, 14) for receiving one or more inputs from a user of the computing device (1).

3. System according to the preceding claim, the at least one input interface (11, 12, 14) being one of a microphone (12), a keyboard, a touch screen (11) and a camera (14).

4. System according to one of claims 2 and 3, the sound played upon reception of the signal representative of the actuation of the sprayer (22) being determined on the basis of the one or more inputs.

5. System according to one of the preceding claims, the switch (23) being an energy harvesting switch for powering the wireless communication interface (20) of the fragrance atomizer (2).

6. System according to one of the preceding claims, the switch (23) and the wireless communication interface (20) of the fragrance atomizer (2) being located in the sprayer (22).

7. Method for creating a multisensory environment with a system according to any one of the preceding claims, said method comprising the steps of:
- actuating by a user the sprayer (22);
- emitting by the fragrance atomizer (2), over the wireless communication interface (20) of the fragrance atomizer (2), a signal indicative of the actuation of the sprayer (22);
- receiving by the computing device (1), over the wireless communication interface (10) of the computing device (1), the signal indicative of the actuation of the sprayer (22);
- playing sound by the computing device (1) upon reception of the signal indicative of the actuation of the sprayer (22).

8. Method according to the preceding claim, further comprising the preliminary steps of:
- receiving by the computing device (1) at least one input from the user indicative of the user's mood and/or personal purpose of the day;
- analyzing by the computing device (1) the received input for determining the user's mood and/or personal purpose of the day;
- selecting by the computing device (1) a sound to be played in said step of playing a sound based on the determined user's mood and/or personal purpose of the day.

9. Method according to the preceding claim, wherein said user input is at least one of a voice sample, a menu selection, a face picture and a text string from the user, or a combination thereof.

10. Computer program containing commands for the computing device (1) to perform the method of one of claims 7 to 9 when the computer program is run on the central processing unit of the computing device (1).
